# EUROPEAN PATENT SPECIFICATION

(11) **EP 0 422 108 B1**
(45) Date of publication and mention of the grant of the patent: **03.08.1994**
(21) Application number: 89908052.7
(22) Date of filing: 30.06.1989
(51) Int. Cl.: A61L 15/00, C09J 153/02

(54) **COMPOSITE ARTICLE RESISTANT TO MOISTURE-INDUCED DEBONDING**
GEGEN DURCH FEUCHTIGKEIT VERURSACHTES ABLÖSEN BESTÄNDIGER VERBUNDKÖRPER
ARTICLE COMPOSITE RESISTANT AU DECOLLAGE INDUIT PAR L'HUMIDITE

(30) Priority: 30.06.1988 US 213789
(43) Date of publication of application: 17.04.1991
(73) Proprietor: H.B. FULLER LICENSING & FINANCING, INC., Wilmington, Delaware 19801 (US)
(72) Inventor: KATSAROS, Mark, G., Mahtomedi, MN 55115 (US); BUNNELLE, William, L., Hugo, MN 55038 (US)
(74) Representative: Draudt, Axel Hermann Christian, Dipl.-Ing.
(86) International application number: US8902882
(87) International publication number: WO9000065

(56) References cited:
- EP-A- 0 156 257
- EP-A- 0 249 979
- GB-A- 1 193 626

## Description

### Field of the Invention

The invention relates to a disposable composite article made by joining its components with an adhesive. The invention more particularly relates to disposable articles made by adhesively bonding a film, a woven or nonwoven fabric, tissue or sheet to a substrate using a novel adhesive that can be sprayed on during construction. Still further, the invention relates to the manufacture of a disposable composite article having a covering envelope and an internal absorbent layer, held within the envelope. The envelope can be formed from a porous front sheet adhered to an impervious back sheet and can have further layers added made of a film or fabric. The absorbent layer can be made of tissue, absorbent fiber, or combinations of other absorbents and wrapping layers. Such layers are constructed using the adhesive of the invention to bind the absorbents in a mechanically stable form or to join the outer wrap to the absorbent. The sprayable hot melt adhesive composition typically contains a particular blend of thermoplastic block copolymer, a compatible tackifying resin, and a solid plasticizer.

More particularly, the invention relates to disposable articles such as disposable infant and adult diapers and feminine pads that are resistant to moisture-induced debonding because of the unique qualities of the adhesives of the invention and to novel methods of article manufacture.

### Background of the Invention

Disposable articles and their manufacture from construction materials including fabrics, films, and adhesives are described in a variety of United States patents. Collins et al., U.S. Pat. No. 4,136,699, teaches a disposable article using a hot melt pressure sensitive adhesive as a positioning and construction material. Such adhesive is typically extruded at high temperature onto the materials of construction during manufacture. Chen et al, U.S. Pat. No. 4,460,364, teaches hot melt pressure sensitive adhesives used in the manufacture of sanitary products. Schmidt, Jr., et al., U.S. Pat. No. 4,526,577, teaches the use of styrene- butadiene-styrene block copolymers in the manufacture of disposable laminates using multi-line extrusion adhesive application technology. Puletti et al., U.S. Pat. No. 4,627,847, also teaches the use of hot melt adhesives in disposable article construction. The adhesives disclosed in Collins et al, Chen et al., Schmidt Jr., et al., and Puletti each use a plasticizing oil, and Puletti teaches that at column 4, lines 1-24, the adhesive can be applied to articles with a variety of conventional methods, including spraying and extrusion.

In conventional production of such articles, hot melt adhesives are typically extruded at elevated temperature directly onto a work piece. Additional layers of a nonwoven fabric or film can be adhered through the hot melt adhesive bonds. With extruded hot melt adhesives, disposable articles with mechanical bonding caused by entrapped layers of fabric or tissue can be made because the adhesive can be extruded at high temperature directly on the work piece. The hot extruded adhesive retains sufficient heat that the material remains liquid for a sufficient time such that the adhesive can soak into the fabric or tissue to form entrapping bonds. Additionally, atomized or misted typically aqueous based adhesives have been used in other end uses in which the adhesive is delivered in the form of a spray of finely divided droplets. However such adhesives have had little or no success in disposable articles.

In recent years, increasing attention has been directed to development of hot melt adhesives that can be sprayed onto the work piece or substrate in a manufacturing regimen. The use of spray-on adhesives have been found to increase productivity. Conventional spray-on adhesives are sprayed from a plurality of narrow orifices in the form of fibers, threads or filaments having a substantially circular cross- section with a diameter of about 0.01 to 0.001 inches. The spray-on adhesive takes on the form of fibers that have substantial surface area in comparison to the mass of the fiber. As a result, the sprayed adhesive fiber cools very rapidly upon contact with the ambient atmosphere. Typically, in the manufacture of disposable articles, after spraying the spray-on adhesives reach ambient temperatures before contacting the work piece or are cooled substantially upon immediate contact with the work piece. This is in sharp contrast to extruded hot melt adhesives that retain a significant amount of heat and can remain melted after application. By ambient temperature, we mean the temperature of the surrounding atmosphere and the temperature of the work piece. In these construction applications, the work piece and the temperature of the manufacturing locus are typically not substantially different. Conventional spray-on adhesives, after application to a work piece, typically take the form of a solid mesh or web which is the result of the combined application of a plurality of adhesive fibers creating a substantially overlapping pattern of threads or fibers of adhesive covering the area of application on the work piece. With conventional spray adhesive technology, the disposable articles are manufactured by contacting a substrate such as a film or woven or nonwoven fabric with the cooled adhesive web on a second substrate and forming a bond between the substrates or layers by pressure. Such conventional spray-on adhesives form typically a laminated surface-to-surface adhesive bond with the film substrates and the fabric layers. By surface-to-surface bonds we mean a physical bond between the adhesive surface and the surface of a sheet or fiber substrate, wherein mechanical entrapment of the substrate in the adhesive does not make a major contribution to bond strength. Conventional spray-on adhesives, when cooled, form adhesive webs with solid characteristics that have little or no cold flow. After cooling, conventional spray-on adhesives have insufficient liquidity to flow onto and wet the surface of the film or fabric or flow into the fabric to entrap or enmesh the fabric in the adhesive mass. Due to the solid nature of the cooled spray-on adhesive, the conventional adhesives rely upon limited surface bonding to obtain an acceptable level of adhesion.

Such surface bonding can result in a disposable article subject to moisture-induced delamination or debonding. The laminated or composite disposable articles often come into contact with moisture from a variety of sources. Such moisture tends to associate with the fibers of the woven or nonwoven fabric or the surface of other substrates. Such moisture can penetrate limited surface-to-surface adhesive bond between the conventional spray-on adhesive and any substrate or porous layer, weakening the bond and resulting in substantial weakening or failure of the bond. These debonding phenomena can be made significantly worse by the hydrophilicity of the underlying substrate. Hydrophilic substrates are preferred since they promote absorbence of moisture in the disposable article. Additionally, in the manufacture of disposable articles, surfactants can be part of any one component or can be post-added to the composite article after manufacture. While such surfactants can improve water absorbence, such surfactants can also enhance moisture-induced delamination. Further, if added to the disposable article after manufacture in the form of an aqueous dispersion or spray, the surfactant can directly enhance moisture-induced delamination.

Such moisture-induced debonding or delamination can be a significant problem in the construction of or the use or disposable diapers, feminine pads, incontinent pads, mattress covers, or any other product in which the article components such as a fabric is joined to a sheet or film substrate through surface adhesive bonds. The physical integrity of the article can be compromised due to adhesive bond failure resulting in waste, inconvenience, embarrassment, or discomfort. We believe that the weakness in the bonds between the fabric and the conventional hot melt adhesives results from the fact that, upon application, the conventional spray-on adhesive cools so rapidly that the adhesive forms only limited surface bond and cannot flow onto the surface or into the fabric to enmesh woven or nonwoven fibers to form a more secure mechanical bond.

Accordingly, a substantial need exists in the manufacture of articles from woven or nonwoven fabrics for spray-on adhesive that retains flowability or liquidity and can resist the moisture-induced delamination or debonding of such articles made with a hot melt spray-on adhesive.

### Brief Discussion of the Invention

We have found a novel spray-on adhesive composition made from components that interact to produce a composition that, after cooling below the softening points of the components, retains sufficient liquidity, for a sufficient period of time, to permit the adhesive composition to flow onto the surface or to penetrate the constituent parts of a disposable composite article, such as a film, a tissue or a woven or nonwoven fabric, sufficiently to enmesh or entrap sufficient fibers and to create a strong mechanical bond that is not substantially weakened, debonded, or delaminated by moisture. In sharp contrast to the limited surface bonding of conventional spray-on adhesives, the spray-on adhesives of the invention do not merely bond to the limited areas of surface of the fibers in the fabric, but cause the adhesive to flow onto the surface to intimately bond or to enmesh or entrap the fibers of the nonwoven. As a result, bonds that involve the mechanical entrapment of the fibers, even in the presence of substantial quantities of moisture, resist weakening, debonding, or delamination. By reciting that the spray-on adhesive of this invention substantially reduces the onset of moisture-induced weakening or delamination, we mean that the adhesive bonds formed by the liquidity of the adhesive retain sufficient bond strength, to prevent failure of the adhesive and to maintain an intact article during use while wet. The intimate bonds formed by the spray-on adhesives of the invention are distinguished from the limited surface bonding present in the prior art by the flowability of the adhesives of the invention. Since the adhesives of the invention retain substantial flowability, bonding is obtained by flowing and fully wetting the surfaces that are combined in the manufacturing processes. In the use of the prior art adhesives, since they become solidified before or immediately after application, they tend to have substantially less flowability and can only bond where applied with little or no cold flow.

### Detailed Description of the Invention

Briefly, the nonwoven articles of the invention obtain their substantial resistance to moisture-induced delamination or debonding because a substrate layer is joined with a separate layer through an adhesive that is sprayed on. The adhesives typically comprise a thermoplastic block copolymer such as an A-B-A block copolymer, an A-(B-A)ₙ-B-A, multi-block or tapered block copolymer, wherein n is an integer of about 1 or more, or a radial block copolymer. In the block copolymer, each A comprises a polystyrene block and each B comprises a rubbery block. In addition to the block copolymer, the adhesive contains a tackifying resin which is a rosin acid or rosin ester or is aliphatic or mixed aliphatic-aromatic in character. The adhesive additionally comprises a plasticizer compound that has a softening point that is greater than disposable article manufacturing ambient temperature (typically 25-35° C.).

We have found that these components cooperate to form an adhesive having improved article assembly properties (low viscosity at application temperatures), but additionally, after spraying and cooling to ambient temperatures, retains a liquid character for a substantial period of time which is sufficient to either fully wet the surface or to penetrate the porous layers. As a result the adhesive retains an extended open time, exhibits a good viscosity profile, and excellent dry and wet bond strength. The adhesive components can be formulated to retain liquid characteristics for a substantial period of time. The adhesive during this liquid period can flow onto the surfaces of the article component to fully wet out the surface creating enhanced bonding resistant to moisture. Additionally, the adhesive during this period of liquidity can flow into, surround, and mechanically entrap or enmesh the fibers of a woven or nonwoven fabric or layer. While solidification is delayed, the adhesive, after solidification occurs, has enhanced cohesive strength because of the nature of the components. The use of the phrase sufficient liquidity for sufficient time to form moisture resistant bonds indicates that the adhesive remains liquid (with a viscosity less than 5,000 cP, preferably less than 2,000 cP, most preferably less than 1,000 cP, at 350° F.) with little or no peel strength (less than 50 grams, preferably less than 30 grams) until solidification occurs over a period typically greater than 5 minutes, greater than 10 minutes or greater than 60 minutes, depending on the adhesive used. Since the adhesive remains liquid and does not solidify it has little peel strength until it solidifies. After solidification the cohesive strength of the adhesive generally exceeds the strength of the materials used in the disposable article.

### The Block Copolymer

Thermoplastic block copolymers that can be used in the novel adhesives of the invention in manufacturing disposable articles are thermoplastic rubbers that terminate in hard, glassy end blocks which are thermodynamically incompatible with the rubbery midblocks. Such polymers consequently consist of two phases in a solid state, a continuous rubber phase and a substantially discontinuous hard, glassy or plastic phase which locks the rubber molecules in place. The end blocks produce physical crosslinking because the end blocks of a plurality of molecules are joined by physical van der Waals attraction in a single domain or crosslinked site. Such an interaction forms domains which are stable. The literature describes numerous molecular variations of the thermoplastic rubber copolymers, including the A-B-A structure, an A-B-C structure, a branched or radial configuration and a multi-block or tapered block structure with repeating segments A-(B-A)ₙ-B-A, and so forth, wherein n is an integer of at least 1 to 15.

The A blocks are typically homopolymeric polystyrene. However, other vinyl arene monomers can be used in preparing either a homo or copolymeric plastic or glassy end or A block. The B blocks typically comprise rubbery polymers derived from diene monomers including isoprene and butadiene. The midblocks can be post treated to improve their heat stability through hydrogenation or other treatment. We believe the size and amount of the A or end blocks in conjunction with the time to reform the end block domains is important to the important properties of the invention. As the A blocks increase in size the rate and tendency of the adhesive to solidify increases. We believe this is due to the interaction between the A blocks, the tackifier and primarily the solid plasticizer. Large A blocks or insufficiently plasticized A blocks can rapidly solidify after cooling, can be free of the delayed solidification effect and can have reduced moisture resistance. While the total styrene content of the polymers can be as much as 51 wt-% of the polymer, and since the polymers can have more than two A blocks for optional performance the largest A block should be less than or equal to about 15 wt-% of the polymer, and most preferably is less than or equal to 10 wt-% of the polymer. In an S-B-S (styrene-butadiene-styrene) copolymer the preferred molecular weight is about 50,000 to 120,000 and the preferred styrene content is about 20 to 35 wt-%. In an S-I-S (styrene-isoprene-styrene) copolymer the preferred molecular weight is about 100,000 to 150,000 and the preferred styrene content is about 14-30 wt-%. Hydrogenating the butadiene midblocks produces rubbery midblocks that are typically considered to be ethylenebutyliene midblocks.

Such block copolymers are available from Shell Chemical Company, Enichem and Fina. Multiblock or tapered block copolymers (the A-(B-A)ₙ-B-A type) are available from Firestone under the STEREON 840A and 845 trademarks.

### Tackifying Resin

The adhesives of the invention contain a tackifying resin in combination with a thermoplastic block copolymer and the plasticizer. Tackifying resins useful in the adhesives of the invention comprise rosin derivatives including wood rosin, tall oil, tall oil derivatives, rosin ester resins, natural and synthetic terpenes and aliphatic or mixed aromatic-aliphatic tackifying resins.

Aromatic monomers useful in forming the aliphatic-aromatic adhesive compositions of the invention can be prepared from any monomer containing substantial aromatic qualities and a polymerizable unsaturated group. Typical examples of such aromatic monomers include the styrenic monomers styrene, alphamethylstyrene, vinyl toluene, methoxystyrene, t-butylstyrene, chlorostyrene, etc., indene monomers including indene, methyl indene, and others. Aliphatic monomers are typically natural and synthetic terpenes which contain C₅ and C₆ cyclohexyl or cyclopenyl saturated groups that can additionally contain a variety of substantially aliphatic ring substituents. Aliphatic tackifying resins can be made by polymerizing a feed stream containing sufficient aliphatic monomer such that the resulting resin exhibits aliphatic characteristics. Such feed streams can contain other aliphatic unsaturated monomers such as 1,3-butadiene, cis-1,3-pentadiene, trans-1,3-pentadiene, 2-methyl-1,3-butadiene, 2-methyl-2-butene, cyclopentadiene, dicyclopentadiene, terpene monomers, and others. Mixed aliphatic-aromatic resins contain sufficient aromatic monomers and sufficient aliphatic monomers and optionally other C₃-C₈ unsaturated monomers to produce a resin having both aliphatic and aromatic character.

The adhesive compositions of the invention can contain rosin and rosin derivatives as a tackifying agent. Rosin is a solid material that occurs naturally in the oleo resin of pine trees and typically is derived from the oleo resinous exudate of the living tree, from aged stumps and from tall oil produced as a by-product of kraft paper manufacture. After it is obtained, rosin can be treated by hydrogenation, dehydrogenation, polymerization, esterification, and others. Rosin is typically classed as a gum rosin, a wood rosin, and as a tall oil rosin. The materials can be used unmodified and additionally can be used in the form of esters of polyhydric alcohols and can be polymerized through the inherent unsaturation of the molecules. The materials are commercially available and can be blended into the adhesive compositions using standard blending techniques.

Representative examples or such rosin derivative tackifying resins include the pentaerythritol esters of tall oil, gum rosin, wood rosin or mixtures thereof.

Representative examples of such aliphatic resins include hydrogenated synthetic C₉ resins, synthetic branched and unbranched C₅ resins and mixtures thereof.

Representative examples of such aromatic aliphatic tackifying resins include styrenated terpene resins, styrenated C₅ resins or mixtures thereof.

The selection of tackifying resins is based on the nature of the B or midblock of the block copolymer. Rosin derivatives are best for S-I-S/S-B-S blends and can be used with S-I-S or S-B-S alone. Hydrogenated C₉ or straight aliphatic resins are preferred for S-I-S copolymers. For S-B-S copolymers styrenated terpenes are preferred.

### Plasticizer

A plasticizer is broadly defined as a typically organic composition that can be added to rubbers and other resins to improve extrudability, flexibility, workability, or stretchability. Typical plasticizers in adhesives are plasticizing oils that are liquid at typical ambient temperature. The plasticizer used in the spray-on adhesives of the invention is typically a solid composition having a softening point of at least 45° C. Preferably, the plasticizer composition has a softening point of at least 60° C. Increased softening points (60-130° C.) can aid in improving heat resistance or preventing bond failure at high temperatures. The selection of plasticizer and the use of small amounts of oil can aid in the control over the time from spraying to solidification. The preferred plasticizers of the invention have some substantial aromatic character. Such aromatic character enables the solid plasticizers to interact with the ackifying resins and the end or A blocks of the thermoplastic copolymer which results in the unique property of the adhesive composition that it retains substantial liquidity for a substantial period of time after cooling below the softening points of the components. The solid plasticizer of the invention retains plasticizing properties during the time it remains in a liquid state prior to adhesive solidification. After solidification the plasticizers can associate with the A or end blocks due to plasticizer aromaticity or can phase out and can no longer interact with and plasticize the polymer units. Once phased out the plasticizer becomes generally inactive with respect to adhesive properties. After the plasticizer solidifies the adhesive retains adhesive and cohesive strength through the polymer and tackifying resin regardless of the physical state of the plasticizer. However the plasticizer, if it does not phase out, can increase cohesive strength of the adhesive after solidification if it associates in the A block phase.

One useful class of plasticizers used in the invention comprises a cyclo-aliphatic or aromatic ester of a benzene dicarboxylic acid. Such plasticizers are prepared by forming an ester from a cyclo-aliphatic or aromatic alcohol such as cyclohexanol, phenol, naphthol, or other monohydroxy alcohol compounds having from 5 to 12 carbon atoms. The ester compounds are formed from dicarboxylic acid compounds, typically phthalic acids. Phthalic acids that can be used in the plasticizers are 1,2-benzene dicarboxylic acid, 1,3-benzene dicarboxylic acid (isophthalic acid), or 1,4-benzene dicarboxylic acid (terephthalic acid). The preferred plasticizers of this class comprise dicyclohexyl phthalate or diphenyl phthalate. Most preferably, dicyclohexyl orthophthalate and is used.

A second class of useful plasticizer comprises an aromatic carboxylic acid ester of a polyfunctional alcohol having 1 to 10 hydroxyl groups. Polyfunctional alcohols that can be used in the compositions of this class of plasticizers include compounds having at least two hydroxyl groups and at least two carbon atoms in the molecule. Specific examples of preferred hydroxy compounds include ethylene glycol, propylene glycol, 1,2-butylene glycol, 1,4-butylene glycol, glycerine, glucose, fructose, sucrose, mannitol, trimethylol ethane, 1,4-cyclohexane dimethanol, pentaerythritol, 2,2-dimethyl-1,3-propane diol, 2-hydroxy methyl-2-methyl-1,3-propane diol, neopentyl glycol, and other useful polyfunctional hydroxyl compounds. Aromatic acids that can be used with the polyfunctional alcohols to form this class ester plasticizer compounds of the invention include aromatic carboxylic acids, typically having at least one aromatic group and at least one carboxyl function. Representative acids include benzoic acid, naphthanoic acid, and 4-methyl benzoic acid. Typical examples of such useful plasticizers include triethylene glycol tribenzoate, trimethylol ethane tribenzoate, 1,4-cyclohexane dimethanol dibenzoate, glycerol tribenzoate, sucrose benzoate, pentaerythritol tetrabenzoate, 2,2-dimethyl-1,3-propane diol dibenzoate, triethylene glycol dibenzoate, glycerol tribenzoate, 2-hydroxymethyl-2-methyl-1,3-propane diol tribenzoate pentaerythrithol tetrabenzoate, neopenyl glycol dibenzoate, mixtures thereof and others.

A third class of useful plasticizers for use in the invention comprise a sulfonamide class made from aromatic sulfonic acids. Such plasticizers generally fall within the structural formula: 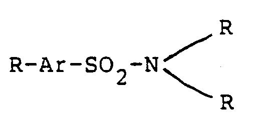
wherein each R is independently selected from the group consisting of hydrogen, aliphatic and cyclo-aliphatic radicals having 1 to 12 carbon atoms. Each R can be typically hydrogen, methyl, ethyl, propyl, isopropyl, n-butyl, isobutyl, tertiary butyl, ethyl hexyl, neopentyl, cyclohexyl, deodecyl, etc. R is preferably methyl, ethyl or cyclohexyl. Such sulfonamide plasticizers can also be used in the form of a resinous material formed through the condensation of formaldehyde with said sulfonamide plasticizer.

In the manufacture of disposable articles, materials or components that are not sufficiently hydrophilic can be treated with surfactants or wetting agents to increase or enhance the wettability or hydrophilicity of the components. Since the disposable articles are designed to absorb and sequester water from the skin of the user of the disposable article, the articles are typically manufactured from hydrophilic materials that absorb and remove water from the surface of the user's skin. However, in certain disposable articles materials that are not sufficiently hydrophilic are used. Such materials include polyolefins, elastics, nonwovens, films, etc. Such materials with measurable hydrophobicity which are not sufficiently hydrophilic can be treated with wetting agents to increase their hydrophilicity. Very often the hydrophobic materials are covers or covering sheets over the hydrophilic materials. To enhance passage of the moisture from the user to the hydrophilic internal layers the hydrophobic cover sheets are made hydrophilic using surfactant or wetting agent materials. Preferred surfactant or wetting materials for use in the invention comprise nonionic surfactants typically manufactured by the oligomerization or polymerization of ethylene oxide or propylene oxide. Such materials typically take the form of polyethylene glycol, polypropylene glycol, ethoxylated or propoxylated alcohols, ethoxylated or propoxylated phenols, or other related molecules having as a major polymer backbone polyethylene oxide, polypropylene oxide, or mixtures thereof.

The adhesive compositions of the invention can contain other compatible polymers, fillers, pigments, dyes, catalysts, inhibitors, antioxidants, UV absorbers, waxes, and other conventional additives.

The following Table A sets forth useful proportions of the components of the invention.

**Table A**

| | Useful | Preferred | Most Preferred |
|---|---|---|---|
| Block copolymer | 10-40 | 12-28 | 15-25 |
| Tackifying resin | 30-80 | 45-75 | 50-65 |
| Plasticizer | 5-40 | 10-30 | 15-25 |

The articles of the invention at a minimum comprise a film layer or a permeable layer adhesively joined with a substrate.

The permeable layer can comprise a cellulosic tissue, a woven or nonwoven fabric or other thin, flexible, porous or wettable sheet-like material. The tissue layer is a well known, typically loosely formed cellulosic sheet of high porosity or permeability. The fabric layer consists of a fluid permeable flexible material that can be made of either hydrophilic or hydrophobic fiber components. Woven and nonwoven webs comprising the fabric can comprise natural or synthetic fibers or mixtures thereof. Woven and nonwoven materials are well known and their construction methods have been practiced for many years. Woven fabrics are typically manufactured in weaving machines forming an interlocking mesh of fibers forming the layer. Nonwoven fabrics can be made through a dry-laid or wet-laid method in carding processes, air laying processes, or spun bond processes to produce a web that is mechanically, chemically, or thermally formed. The fabric layers for use in the compounds and articles of this invention typically have a basis weight in the range of about 10 to 25, preferably 14 to 18 grams per square yard, a minimum dry tensile strength of at least 800 grams per centimeter² squared in the machine direction, and at least 200 grams per centimeter² in a cross machine direction. Synthetic materials commonly used in forming the fabric top sheets include rayon, polyester, polypropylene, polyethylene, nylon, and others.

The substrate materials that can be used in the manufacture of the disposable articles of the invention, in combination with the tissue or woven or nonwoven fabric, comprises any typical substrate used in the manufacture of disposable articles including films, sheets, elastics, absorbents, cellulosic fluffs or fill, other tissue, woven or nonwoven fabrics, etc.

Absorbent layers can be adhered to other substrates using the adhesives of the invention. Such absorbent layers can comprise cellulosic pulp or fluff. Such fluff layers are often formed and wrapped in tissue to provide mechanical integrity to the fluff which has little inherent integrity. Fluff is typically manufactured through formation of cellulosic fibers. However, other materials can be utilized to form high absorbent fluff or pulp layers.

Elastic bands or elements can be used in the manufacture of the disposable articles of this invention.

The film or sheet-like layer used in the invention comprises a flexible sheet-like or film substrate. Such films are typically manufactured from thermoplastic resins and take the form of a thin layer having a thickness of about 0.5 to 2.0 mils. Such films comprise polyethylene, polypropylene, ethylene-propylene copolymers, ethylene acrylate copolymers, ethylene vinyl acetate copolymers, polyvinyl chloride polymers, polyvinylidene chloride polymers, polyester polymers, and others. Such films can be perforate or imperforate. In addition to the above materials used in the composite articles of the invention, a variety of other materials can be used, including other wrapping materials, deodorants, perfumes, dyes, and decorative appliques, which provide further absorbency, instructional legends, and pleasing appearance or smells.

In somewhat greater detail, the adhesives of the invention can be used in the manufacture of disposable articles including disposable diapers, incontinent devices or diapers, feminine pads, and disposable bed pads by adhering a porous layer to a substrate. The assembly operations that deserve note include adhering a porous nonwoven layer to a back sheet and adhering a tissue layer to an absorbent core.

In the manufacture of absorbents for disposables, it is common to wrap loosely assembled fluff or batts of absorbent material within a tissue overwrap. In such manufacture, the tissue surrounds the absorbent material in an overlapping fashion such that the spray-on adhesive can be applied to the overlap area, causing the adhesive to penetrate the overlap to contact the underlying fluff or batt. The spray-on adhesive in contact with the tissue and absorbent material forms a strong mechanical bond which maintains the tissue wrap and provides mechanical support and integrity to the underlying fluff or absorbent batt material. As a result of using the manufacturing techniques of the invention, the tissue-covered absorbent material obtains substantial mechanical integrity from the adhesive and tissue structure. During use, the tissue and adhesive maintains the fluff or batt in place and prevents movement of the absorbent material resulting in an inappropriate segregation of absorbent material in a small portion of the absorbent article. Such mechanical integrity insures that the absorbent material stays in place to provide absorbency and protection.

In the manufacture of composite articles, the fluid permeable fabric top sheet is adhered to a film back sheet. An absorbent layer can be introduced into the space between the fabric layer and the back sheet. Typically a fluid in contact with the fabric layer passes through the fabric layer and is absorbed and held within the absorbent layer. The absorbent core typically comprises a highly porous, highly absorbent loosely contacted fluff, wrapped or encased within a tissue cover. The absorbent fluff typically has little mechanical integrity. The tissue wrap or cover, once adhered to the fluff, provides the absorbent layer with substantial dimensional integrity preventing the absorbent material from migrating or collecting in an inappropriate portion of the diaper. The tissue wrap ensures that the absorbent material remains evenly distributed within the envelope created by the back sheet and the fabric layer. The manufactured diaper or the components of the diaper can have elastic bands or segments adhesively attached to provide security for the wearer. Such elastic bands create a snug fit at the waist and the leg apertures of the disposable articles. The adhesive compositions of the invention can be used to form bonds between the surfaces of the film materials between apertured films and nonapertured films, between tissue and nonwoven or woven fabric layers, between absorbent fluff and tissue overwraps, and between elastic bands or elements and any structural component of the disposable diaper.

In construction methods for the preparation of the disposable articles of the invention, the adhesives are typically applied from spray heads that deliver the adhesive at elevated temperatures (typically above about 250°F. and typically in the range of 275-400°F.). The spray heads have apertures that range from about 0.01 to about 0.04 inches. Under the operating conditions of typical adhesive spray machines, the diameter of the sprayed adhesive fiber can range from the size of the aperture to as little as about 0.001 inches depending on operating conditions. Depending on the end use and final bond strength desired, the adhesive can be used at application amounts that range from 0.5 milligrams per square inch to as much as 10 milligrams per square inch. Preferably, because of the unique properties of the adhesives of this invention, the adhesives can be used at an application amount of from about 0.5 milligrams per square inch to 5 milligrams per square inch. Conventional spray-on adhesives are typically used at high add-on rates within a range of 6 to 7 milligrams per square inch because of their tendency to debond or delaminate in the presence of moisture. The conventional add-on rate appears to be excessive because bonding characteristics of the adhesives are poor. Most preferably, in disposable diaper construction the adhesive of the invention is used at an application rate of about 1 to about 4 milligrams per square inch.

During the manufacture of disposable articles using the adhesives of the invention, two modes of application are preferred. One mode of operation involves spraying the adhesive upon a fabric, such as a tissue, a woven or nonwoven web, or other material having permeability to the adhesive. Such sprayed-on adhesive can penetrate the permeable tissue, nonwoven or woven fiber, to cause the sheet to be embedded in the adhesive and adhered to the substrate such as an absorbent layer, back layer, or film. Alternatively, the adhesives of the invention can be directly applied to back sheet or film and the tissue, woven or nonwoven fabric, or other material can be applied to the adhesive on the film. The adhesive retains sufficient liquidity that it can penetrate pores or apertures in the fabric to form a mechanical bond. In the manufacture of tissue fluff absorbent cores, the fluff is typically wrapped by tissue. The tissue layer can be wrapped around the fluff and can overlap. Adhesive can then be sprayed on the overlapping portion of tissue outerwrap, can penetrate the wrappings and adhere the tissue to the fluff ensuring that the fluff obtains dimensional stability from adherence to the outer wrap.

In somewhat greater detail, the sprayable, hot melt adhesive compositions of the invention typically comprise an effective amount of a thermoplastic block copolymer base and an effective amount of a tackifying agent and sufficient solid plasticizers to form an effective adhesive that has the unique property that after spraying and cooling retains sufficient liquidity to penetrate a porous layer.

The hot melt adhesives of the invention are made in common hot melt manufacturing equipment. In the manufacture of the hot melt adhesives of the invention, the thermoplastic block copolymers typically added to a melt comprising either the tackifier or the plasticizer material or mixtures thereof. Such additions facilitate the blending of the block copolymer into a smooth, uniform mixture. In such a manufacturing regimen, either the tackifier or the plasticizer or a portion thereof is added to the manufacturing equipment under inert atmosphere and is heated and agitated until melted. The thermoplastic block copolymer is then added to the melt at a rate such that the mixture forms a uniform smooth blend within a reasonable period. Antioxidant materials used in the manufacture of the adhesive can be added to the melt prior to, with, or after the addition of the block copolymer. Once a smooth blend of the copolymer in conjunction with an adhesive component is formed, the balance of the components of the hot melt adhesives can be added at a convenient rate. Once the uniform blend of all the adhesive ingredients is formed, the adhesive can be drawn off and packaged in a convenient form including in drums, blocks, pillows, pellets, granules, etc.

The following examples provide additional information with respect to the manufacture of the adhesives of the invention and include the best mode.

### Example I

Into a sigma blade mixer having a nitrogen atmosphere and heated to a temperature of 350° F. was added about 170 grams of styrenated aliphatic tackifying resin (WINGTACK EXTRA, Goodyear) and 8.5 grams of an antioxidant (ETHYL 330, Ethyl Corp.). The mixer was operated until the antioxidant was fully blended with the molten tackifying resin. Into the tackifying resin was added 170 grams of a styrene-isoprene-styrene block copolymer having 17 wt-% styrene (KRATON D-1117, Shell Chemical Co.). After the copolymer had been fully added to the resin and a uniform melt resulted, an additional 331.5 grams of the tackifying resin was slowly added to the mixer followed by 170 grams of a dicyclohexylphthalate plasticizer (MORFLEX 150, Pfizer). The single blade mixer was continued until the contents were a smooth mel blend and the material was withdrawn and packaged.

### Example II

Following the procedure of Example I the following compositions were blended into a hot melt adhesive:

| Ingredient | Trade Name | Grams |
|---|---|---|
| S-B-S/S-I-S blend | KRATON 1102 & KRATON 1117 | 5/14 |
| Antioxidant | IRGANOX 1330 | 1 |
| Tackifying agent | PERMALYN 305 | 59 |
| Dicyclohexylphthalate plasticizer | MORFLEX 150 | 21 |

### Example III

Following the procedure of Example I the following compositions were blended into a hot melt adhesive:

| Ingredient | Trade Name | Grams |
|---|---|---|
| S-B-S block copolymer | KRATON 1102 | 15 |
| Tackifier | PERMALYN 305 | 59 |
| Antioxidant | ETHANOX 330 | 1 |
| Dicyclohexylphthalate plasticizer | MORFLEX 150 | 25 |

### Example IV

Following the procedure of Example I the following compositions were blended into a hot melt adhesive:

| Ingredient | Trade Name | Grams |
|---|---|---|
| Styrenated terpene tackifying resin | ZONATAC 501 Light | 59 |
| S-I-S/S-B-S blend polymer | KRATON 1117 & KRATON 1102 | 14/5 |
| Antioxidant | ETHANOX 330 | 1 |
| Dicyclohexylphthalate plasticizer | MORFLEX 150 | 21 |

### Example V

| Ingredient | Trade Name | Grams |
|---|---|---|
| Tapered block copolymer | STEREON 840A | 19.7 |
| Styrenated terpene resin | ZONATAC 501 | 56.0 |
| Antioxidant | IRGANOX 1076/ Butyl Zimate | 0.55/ 0.55 |
| DCHP | MORFLEX 150 | 23.2 |

### Example VI

| Ingredient | Trade Name | Grams |
|---|---|---|
| S-I-S block copolymer | KRATON-1117 | 20.0 |
| Tackifier | PERMALYN 305 | 59.8 |
| 1,4-cyclohexane dimethanol dibenzoate | BENZOFLEX 352 | 20.0 |
| Antioxidant | IRGANOX 1010 | 0.2 |

### Comparative Example A

Following the procedure of Example I, 155 grams of a hydrogenated C₉ tackifying resin (ESCOREZ 5320) and 25.5 grams of a 50-50 blend of antioxidants (IRGANOX 1010 and IRGANOX 1076) was blended in the sigma mixer followed by 127.5 grams of a styrene-ethylene-butylene-styrene block copolymer having 30 wt-% styrene (KRATON G 1652, Shell Chemical Co.). 309.95 grams of the tackifying resin was then blended into the melt followed by 255 grams of a naphthenic oil (SHELLFLEX 371).

### Comparative Example B

Following the procedure of Comparative Example A the following compositions were blended into a hot melt adhesive:

| Ingredient | Trade Name | Grams |
|---|---|---|
| Atactic poly alpha olefin | B3A15 | 425 |
| Dicyclohexylphthalate plasticizer | DCHP | 85 |
| Tackifying resin | WINGTACK EXTRA | 318.75 |
| Antioxidant | IRGANOX 1010 & IRGANOX 1076 | 4.25 |
| Polyethylene | EPOLENE C10 | 17.0 |

### Comparative Example C

Same as Comparative Example A except Shellflex oil was omitted and 549.95 grams of tackifying resin and 170 grams of DCHP were used.

### Comparative Example D

Same as Example V except DCHP was omitted and an equal amount of a plasticizing oil (1200 ACS) was used.

### Moisture Resistance Test for Spray Applied Hot Melt Adhesives

The test method is intended to assist in determining the differences in wet and dry bond strengths of spray applied hot melt adhesives.

### Equipment

1. High quality hot melt spray equipment intended for the nonwoven industry. Preferred equipment manufacturers are Nordson, Acumeter, Slautterback, and Mercer.
2. Tensile tester or T-peel tester with a load range of 1,000 grams and capable of a grip separation rate of 6 inches/minute.
3. Cutting board or 1" x 6" cutting template.
4. Suitable substrates.
5. Purified water.

### Sample Preparation

1. Spray apply the adhesive to one substrate at the rate of 4 mg./sq. in. Quickly nip the second substrate to the adhesive using a nip pressure of 1.4 (20 psi). If the bond is expected to give substrate failure, slip in 5-10 pieces of 2" wide release paper across the bond line.
   Make sure the spray pattern is as even as possible since small variations in coat weight can cause unexpectedly large variations in peel values.
2. Cut a 1" x 6" sample out of the laminated area in the machine direction. If release paper was used, cut it in half in the cross direction and then cut 6" strips in the machine direction. Peel back 1" (or use the 1" release paper edge) and apply staples to both substrates to assist gripping. Make ten samples.
3. Condition the samples at room temperature and 50+/- 10% RH for at least 16 hours, but not more than 30 hours.

### Test Procedure

1. Set the peel tester to the gram scale 6 inches/minute, 25 seconds.
2. Dry delamination - clamp the substrates into the peel tester. Generally, the weaker substrate will be clamped to the moving grips. Set the position so that there is tension on the adhesive. Turn the peel tester on. Peel a total of five samples.

Wet Delamination - place the 6" sample vertically into five inches of water. Leave in water for 30 seconds (longer if the substrates do not readily absorb water). Pat dry with paper towels. Attach the substrates to the clamps as described above. Set the position so that there is tension on the adhesive. Turn the peel tester on. Peel a total of five samples.

### Report

Average peel strength of the dry and wet samples. Note the type of failure that occurred. Also, report coat weight, application temperature, substrates used, and spray equipment used. Also note if water soak was longer than specified, or if more time was necessary after application to allow the adhesive to recrystallize completely.

| Test Results | | |
|---|---|---|
| Example A: | Dry: | good pull; some fiber tear. |
| | Wet: | adhesive failure. |
| Example B: | Dry: | weak bond. |
| | Wet: | no bond. |
| Example C: | Dry: | no bond to substrate |
| | Wet: | no bond to substrate. |
| Example D: | Dry: | substrate failure. |
| | Wet: | 34 grams. |
| Example I: | Dry: | fiber tearing bond. |
| | Wet: | resistance to debonding seen. |
| Example II: | Dry: | fiber tearing bond (better than Example I) |
| | Wet: | tissue failure - no bond failure (better than Example I). |
| Example III: | Dry: | good bond - slight tissue failure. |
| | Wet: | good bond |
| Example IV: | Dry: | initial - substrate failure; at 24 hours - substrate failure. |
| | Wet: | initial 34 grams; at 24 hours -substrate failure. |
| Example V: | Dry: | substrate failure. |
| | Wet: | substrate failure. |
| Example VI: | Dry: | cohesive failure if pulled slowly -tissue failure if pulled fast; |
| | Wet: | excellent bond tissue failure - wet with nonionic surfactant - weaker bonds but tissue failure if pulled fast. |

The Examples and data shown above indicate that the use of a solid plasticizer such as dicyclohexylphthalate in combination with a particular selection of block copolymer and tackifying resin can produce an adhesive having both dry and wet bond strength to both film and porous substrate. The comparative Examples indicate that polymers that are not block copolymers and adhesives containing plasticizers other than those similar in properties to dicyclohexylphthalate do not possess significant wet strength, and in some cases have poor dry strength. Additionally we have found that a blend of S-I-S and S-B-S copolymers can, in certain formulae, provide enhanced wet and dry bonding properties.

Since many embodiments of the invention set forth above can be made without departing from the spirit and scope of the invention, the above specification, Examples and data are non-limiting and the invention resides in the claims hereinafter appended.

## Claims

1. A sprayable hot melt adhesive composition having superior wet bond strength, said adhesive comprising:
(a) about 10-40 parts by weight of a thermoplastic block copolymer selected from the group consisting of an A-B-A block copolymer, an A-(B-A)ₙ-B-A block copolymer, wherein n is an integer of about 1 or more, and a radial block copolymer, each A comprising a polystyrene block and each B comprising a rubbery block;
(b) about 30-80 parts by weight of a tackifying rosin derivative, an aliphatic tackifying resin, a mixed aliphatic-aromatic tackifying resin or mixtures thereof; and
(c) about 5-40 parts by weight of a plasticizer compound that has a softening point of above about 45° C; and wherein, after the adhesive is sprayed on and cooled to ambient temperatures, the adhesive retains sufficient liquidity for a sufficient time to wet or to penetrate a substrate or layer to form a moisture-resistant bond.

2. The adhesive of claim 1 wherein the adhesive when cooled obtains a peel strength of less than 50 grams for at least 5 minutes and thereafter obtains a peel strength in excess of 100 grams.

3. The adhesive of claim 1 or 2 wherein the thermoplastic block copolymer comprises an A-B-A block copolymer having a polyisoprene midblock with a molecular weight of about 100,000 to 150,000 and a styrene content of about 14 to 30 wt-%, the tackifying resin being preferably an aliphatic tackifying resin;
or the thermoplastic block copolymer composition comprises an A-B-A block copolymer having polybutadiene midblock with a molecular weight of about 50,000 to 120,000 and a styrene content about 20 to 35 wt-%, the tackifying agent preferably being a styrenated terpene;
or the block copolymer comprises a blend of a styrene-isoprene-styrene polymer and a styrene-butadiene-styrene copolymer at a weight ratio of about 2 to 5:1, the plasticizer preferably comprising an aromatic sulfonamide, and
the tackifier preferably comprising a wood rosin derivative, especially a pentaerythritol ester of a rosin acid.

4. The adhesive of claim 1 wherein the tackifying resin is an aliphatic tackifying resin, especially an aliphaticaromatic tackifying resin.

5. The adhesive of any one of claims 1-4 wherein the plasticizer has a softening point of at least about 65° C.

6. The adhesive of any one of claims 1-5 wherein the plasticizer comprises an aliphatic, especially cycloaliphatic or aromatic ester of a benzene dicarboxylic acid, especially a 1,2-benzene dicarboxylic acid, the plasticizer preferably being dicyclohexyl phtalate.

7. The adhesive of any one of claims 1-5 wherein the plasticizer comprises an aromatic carboxylic acid ester of a polyhydroxy compound having 1 to 10 hydroxyl groups, especially an ester of ethylene glycol, glycerine, neopentyl glycol, pentaerithritol, glucose, sucrose, or mixtures thereof, and benzoic acid or 4-methyl-benzoic acid.

8. The adhesive of any one of claims 1-5 wherein the plasticizer comprises a tri-substituted phosphate or an aromatic sulfonamide.

9. The adhesive of any one of claims 1-8 which additionally comprises a surfactant, especially a nonionic surfactant.

10. The adhesive of any one of claims 1-9, comprising:
(a) about 10 to 40 parts by weight of a thermoplastic A-B-A block copolymer wherein each A comprises a polystyrene block and each B comprises a rubbery butadiene block having a molecular weight of about 50,000 to 120,000 and about 20 to 35 wt-% styrene or a rubbery isoprene block having a molecular weight of about 100,000 to 150,000 and about 14 to 30 wt-% styrene;
(b) about 30 to 80 parts by weight of a rosin ester; and
(c) about 5 to 40 parts by weight of an aromatic plasticizer comprising dicyclohexyl phtalate or 1,4-dicyclohexane dimethanol dibenzoate;
wherein the sprayed-on adhesive after cooling retains sufficient liquidity to fully wet or penetrate a substrate.

11. An article resistant to moisture-induced debonding comprising a substrate joined with a second layer by a sprayed-on adhesive according to any one of claims 1-10 wherein, after the adhesive is sprayed on and cooled to ambient temperature, the adhesive retains sufficient liquidity for a sufficient time to wet or to penetrate the substrate or the second layer to form a moisture-resistant bond.

12. The article of claim 11 wherein the substrate comprises a film, and the second layer comprises a nonwoven fabric, and the fabric is entrapped by the adhesive, the substrate film preferably comprising a polyethylene film, a polypropylene film, a nylon film, a polyvinylidene chloride film, a polyvinyl chloride film, or a polyester film

13. The article of claim 11 wherein the substrate is a mass of absorbent fiber, the second layer is a tissue over-wrap, and both the substrate and the second layer are entrapped by the adhesive.

14. A disposable diaper according to claim 11 or 12, comprising a nonwoven layer bonded to a film layer through a sprayed on adhesive, said disposable diaper resistant to moisture induced debonding, said adhesive comprising:
(a) about 10 to 40 parts by weight of a thermoplastic A-B-A block copolymer, wherein each A comprises a polystyrene block and B comprises a rubbery butadiene midblock, wherein the molecular weight of the polymer comprises 50,000 to 120,000 and the polymer comprises 20 to 35 wt-% polystyrene or a rubbery isoprene midblock wherein the molecular weight of the pomymer comprises 100,000 to 150,000 and about 14 to 30 wt-% styrene;
(b) about 30 to 80 parts by weight of an aliphatic or aliphatic-aromatic tackifying resin; and
(c) about 5 to 40 parts by weight of an aromatic plasticizer compound comprising (i) a cycloaliphatic or aromatic ester of a 1,2-benzene dicarboxylic acid compound or (ii) a benzoic acid ester of a C₂₋₁₂ aliphatic or cyclo-aliphatic diol having a softening point above about 45° C, especially 1,4-cyclohexane dimethanol dibenzoate,
wherein after cooling to ambient temperature, the sprayed on adhesive retains sufficient liquidity to penetrate the nonwoven layer and at least some portion of the nonwoven layer is entrapped by and embedded in the spray-on adhesive.

15. An absorbent inner layer, for a disposable diaper, resistant to moisture induced debonding, said layer comprising a fibrous absorbent cellulosic mass having a tissue overwrap bonded to the cellulosic mass with an adhesive according to any one of claims 1-10 sprayed on the tissue overwrap, said adhesive comprising:
(a) about 10 to 40 parts by weight of a thermoplastic A-B-A block copolymer wherein each A comprises a polystyrene block and each B comprises a rubbery butadiene midblock having a molecular weight of about 50,000 to 120,000 and about 20 to 35 wt-% styrene or a rubbery isoprene block having a molecular weight of about 100,000 to 150,000 and about 14 to 30 wt-% styrene;
(b) about 30 to 80 parts by weight of a tackifying rosin derivative, an aliphatic tackifying resin or a mixed aliphatic-aromatic tackifying resin, especially a styrenated-terpene tackifier; and
(c) about 5 to 40 parts by weight of an aromatic plasticizer comprising (i) a cyclic aliphatic alcohol, especially cyclohexanol, or an aromatic alcohol, especially phenol, ester of a benzene dicarboxylic acid compound, especially a 1,2-benzene dicarboxylic acid, or (ii) a benzoic acid ester of a C₂₋₁₂ aliphatic or cyclic aliphatic diol, having a softening point above about 45° C;
wherein the sprayed on adhesive after cooling retains sufficient liquidity for at least 5 minutes to penetrate the tissue overwrap and bond to the cellulosic mass.

## Patentansprüche

1. Eine sprühbare Heißschmelzklebstoff-Zusammensetzung mit hervorragender Naß-Haftfestigkeit, wobei der Klebstoff umfaßt:
(a) ungefähr 10-40 Gewichtsteile eines thermoplastischen Blockcopolymers, ausgewählt aus der Gruppe, die aus einem A-B-A-Blockcopolymer, einem A-(B-A)ₙ-B-A-Block-copolymer, wobei n eine ganze Zahl von mindestens 1 ist, und einem radialen Blockcopolymer besteht, wobei jedes A einen Polystyrolblock umfaßt und jedes B einen gummiartigen Block umfaßt;
(b) ungefähr 30-80 Gewichtsteile eines klebrigmachenden Kolophoniumderivates, eines aliphatischen klebrigmachenden Harzes, eines gemischt aliphatischaromatischen klebrigmachenden Harzes oder Mischungen dieser; und
(c) ungefähr 5-40 Gewichtsteile einer weichmachenden Verbindung, die eine Erweichungstemperatur oberhalb ca. 45°C hat;
und wobei der Klebstoff, nachdem er aufgesprüht und auf Umgebungstemperatur abgekühlt ist, über einen ausreichenden Zeitraum ausreichende Fließfähigkeit beibehält, um ein Substrat oder eine Schicht zur Bildung einer feuchtigkeitsbeständigen Bindung zu benetzen oder zu durchdringen.

2. Der Klebstoff gemäß Anspruch 1, wobei der Klebstoff, wenn abgekühlt, für mindestens 5 Minuten eine Schälfestigkeit von weniger als 50 Gramm erreicht und danach eine Schälfestigkeit von über 100 Gramm erreicht.

3. Der Klebstoff gemäß Anspruch 1 oder 2, wobei das thermoplastische Blockcopolymer ein A-B-A-Blockcopolymer mit einem Polyisopren-Mittelblock umfaßt, das ein Molekulargewicht von ca. 100.000 bis 150.000 und einen Styrolgehalt von ca. 14 bis 30 Gew.-% hat, wobei das klebrigmachende Harz vorzugsweise ein aliphatisches klebrigmachendes Harz ist;
oder die thermoplastische Blockcopolymerzusammensetzung ein A-B-A-Blockcopolymer mit einem Polybutadien-Mittelblock umfaßt, das ein Molekulargewicht von ca. 50.000 bis 120.000 und einen Styrolgehalt von ca. 20 bis 35 Gew.-% hat, wobei das klebrigmachende Mittel vorzugsweise ein styrolisiertes Terpen ist;
oder das Blockcopolymer eine Mischung aus einem Styrol-Isopren-Styrol-Polymer und einem Styrol-Butadien-Styrol-Copolymer in einem Gewichtsverhältnis von ca. 2 bis 5:1 umfaßt, wobei der Weichmacher vorzugsweise ein aromatisches Sulfonamid umfaßt, und
der Klebrigmacher vorzugsweise ein Wurzelkolophoniumderivat, insbesondere einen Pentaerythritolester einer Kolophoniumsäure, umfaßt.

4. Der Klebstoff gemäß Anspruch 1, wobei das klebrigmachende Harz ein aliphatisches klebrigmachendes Harz, insbesondere ein aliphatisch-aromatisches klebrigmachendes Harz ist.

5. Der Klebstoff gemäß irgendeinem der Ansprüche 1 bis 4, wobei der Weichmacher eine Erweichungstemperatur von mindestens ca. 65°C hat.

6. Der Klebstoff gemäß irgendeinem der Ansprüche 1 bis 5, wobei der Weichmacher einen aliphatischen, insbesondere einen cycloaliphatischen oder aromatischen Ester einer Benzoldicarbonsäure, insbesondere einer 1,2-Benzoldicarbonsäure umfaßt, wobei der Weichmacher vorzugsweise Dicyclohexylphthalat ist.

7. Der Klebstoff gemäß irgendeinem der Ansprüche 1 bis 5, wobei der Weichmacher einen aromatischen Carbonsäureester einer Polyhydroxyverbindung mit 1 bis 10 Hydroxylgruppen, insbesondere einen Ester aus Ethylenglycol, Glycerin, Neopentylglycol, Pentaerythritol, Glucose, Sucrose, oder Mischungen dieser und Benzoesäure oder 4-Methylbenzoesäure umfaßt.

8. Der Klebstoff gemäß irgendeinem der Ansprüche 1 bis 5, wobei der Weichmacher ein trisubstituiertes Phosphat oder ein aromatisches Sulfonamid umfaßt.

9. Der Klebstoff gemäß irgendeinem der Ansprüche 1 bis 8, der zusätzlich eine oberflächenaktive Substanz, insbesondere eine nichtionische oberflächenaktive Substanz umfaßt.

10. Der Klebstoff gemäß irgendeinem der Ansprüche 1 bis 9, umfassend:
(a) ungefähr 10 bis 40 Gewichtsteile eines thermoplastischen A-B-A-Blockcopolymers, wobei jedes A einen Polystyrolblock umfaßt und jedes B einen gummiartigen Butadienblock, wobei das Polymer ein Molekulargewicht von ca. 50.000 bis 120.000 hat und ca. 20 bis 35 Gew.-% Styrol enthält, oder einen gummiartigen Isoprenblock, wobei das Polymer ein Molekulargewicht von ca. 100.000 bis 150.000 hat und 14 bis 30 Gew.-% Styrol enthält, umfaßt;
(b) ungefähr 30 bis 80 Gewichtsteile eine Kolophoniumesters; und
(c) ungefähr 5 bis 40 Gewichtsteile eines aromatischen, Dicyclohexylphthalat oder den Dibenzoesäureester von 1,4-Cyclohexandimethanol umfassenden Weichmachers;
wobei der aufgesprühte Klebstoff nach dem Abkühlen ausreichende Fließfähigkeit beibehält, um ein Substrat vollkommen zu benetzen oder zu durchdringen.

11. Ein Artikel, der gegen feuchtigkeitsinduzierte Entklebungsvorgänge beständig ist, umfassend ein Substrat, das durch einen aufgesprühten Klebstoff gemäß irgendeinem der Ansprüche 1 bis 10 mit einer zweiten Schicht verbunden ist, wobei der Klebstoff, nachdem er aufgesprüht und auf Umgebungstemperatur abgekühlt ist, über einen ausreichenden Zeitraum ausreichende Fließfähigkeit beibehält, um das Substrat oder die zweite Schicht zur Bildung einer feuchtigkeitsbeständigen Bindung zu benetzen oder zu durchdringen.

12. Der Artikel gemäß Anspruch 11, wobei das Substrat einen Film und die zweite Schicht einen vom Klebstoff eingebundenen Faservliesstoff umfaßt, wobei der Substratfilm vorzugsweise einen Polyethylenfilm, einen Polyprophylenfilm, einen Nylonfilm, einen Polyvinylidenchloridfilm, einen Polyvinylchloridfilm oder einen Polyesterfilm umfaßt.

13. Der Artikel gemäß Anspruch 11, wobei das Substrat eine Masse aus absorbierenden Fasern ist, die zweite Schicht eine Umhüllung aus Vliesstoff ist und sowohl das Substrat als auch die zweite Schicht von dem Klebstoff eingebunden sind.

14. Eine Wegwerfwindel gemäß Anspruch 11 oder 12, die gegen feuchtigkeitsinduzierte Entklebungsvorgänge beständig ist, umfassend eine durch einen aufgesprühten Klebstoff an eine Filmschicht gebundene Faservliesstoffschicht, wobei der Klebstoff umfaßt:
(a) ungefähr 10 bis 40 Gewichtsteile eines thermoplastischen A-B-A-Blockcopolymers, wobei jedes A einen Polystyrolblock umfaßt, und B einen gummiartigen Butadien-Mittelblock, wobei das Polymer ein Molekulargewicht von 50.000 bis 120.000 hat und 20 bis 35 Gew.-% Polystyrol enthält, oder einen gummiartigen Isopren-Mittelblock, wobei das Polymer ein Molekulargewicht von 100.000 bis 150.000 hat und ca. 14 bis 30 Gew.-% Styrol enthält, umfaßt;
(b) ungefähr 30 bis 80 Gewichtsteile eines aliphatischen oder aliphatisch-aromatischen klebrigmachenden Harzes; und
(c) ungefähr 5 bis 40 Gewichtsteile einer aromatischen weichmachenden Verbindung, umfassend (i) einen cycloaliphatischen oder aromatischen Ester einer 1,2-Benzoldicarbonsäureverbindung oder (ii) einen Benzeosäureester eines aliphatischen oder cycloaliphatischen C₂₋₁₂-Diols mit einer Erweichungs-temperatur oberhalb ungefähr 45°C, insbesondere den Dibenzoesäureester von 1,4-Cyclohexandimethanol,
wobei der aufgesprühte Klebstoff, nach dem Abkühlen auf Umgebungstemperatur, ausreichende Fließfähigkeit beibehält, so daß er die Faservliesstoffschicht durchdringt und zumindest ein Teilbereich der Faserfließstoffschicht in den Aufsprühklebstoff eingebettet wird.

15. Eine absorbierende Innenschicht für eine Wegwerfwindel, die gegen feuchtigkeitsinduzierte Entklebungsvorgänge beständig ist, wobei die Schicht eine faserstoffartige, absorbierende Cellulosemasse umfaßt, die eine Vliesstoffumhüllung aufweist, die mittels eines auf die Vliesstoffumhüllung aufgesprühten Klebstoffs gemäß irgendeinem der Ansprüche 1 bis 10 mit der Cellulosemasse verbunden ist, wobei der Klebstoff umfaßt:
(a) ungefähr 10 bis 40 Gewichtsteile eines thermoplastischen A-B-A-Blockcopolymers, wobei jedes A einen Polystyrolblock umfaßt und jedes B einen gummiartigen Butadien-Mittelblock, wobei das Polymer ein Molekulargewicht von ca. 50.000 bis 120.000 hat und ca. 20 bis 35 Gew.-% Styrol enthält, oder einen gummiartigen Isoprenblock, wobei das Polymer ein Molekulargewicht von ca. 100.000 bis 150.000 hat und ca. 14 bis 30 Gew.-% Styrol enthält, umfaßt;
(b) ungefähr 30 bis 80 Gewichtsteile eines klebrigmachenden Kolophoniumderivates, eines aliphatischen klebrigmachenden Harzes oder eines gemischt aliphatisch-aromatischen klebrigmachenden Harzes, insbesondere eines styrolisierten Terpen-Klebrigmachers; und
(c) ungefähr 5 bis 40 Gewichtsteile eines aromatischen Weichmachers, umfassend (i) einen Ester aus einem cyclischen aliphatischen Alkohol, insbesondere Cyclohexanol, oder einem aromatischen Alkohol, insbesondere Phenol, und einer Benzoldicarbonsäureverbindung, insbesondere einer 1,2-Benzoldicarbonsäure oder (ii) einen Benzoesäureester eines aliphatischen oder cycloaliphatischen C₂₋₁₂-Diols mit einer Erweichungstemperatur oberhalb ungefähr 45°C;
wobei der aufgesprühte Klebstoff nach Abkühlung über mindestens 5 Minuten ausreichende Fließfähigkeit beibehält, um die Vliesstoffumhüllung zu durchdringen und an die Cellulosemasse zu binden.

## Revendications

1. Composition d'adhésif thermofusible à pulvériser, ayant une adhésivité supérieure à l'état humide, ledit adhésif comprenant :
(a) environ 10-40 Parties en poids d'un copolymère séquencé thermoplastique choisi dans le groupe constitué par un copolymère séquencé A-B-A, un copolymère séquencé A-(B-A)ₙ-B-A, dans lequel n est un nombre entier d'environ 1 ou plus, et un copolymère séquencé radial, chaque A comprenant un bloc de polystyrène et chaque B comprenant un bloc caoutchouteux ;
(b) environ 30-80 parties en poids d'un dérivé de colophane poissant, d'une résine poissante aliphatique, d'une résine poissante mixte aliphatique-aromatique, ou de mélanges de ceux-ci ;
(c) environ 5-40 parties en poids d'un composé plastifiant ayant un point de ramollissement supérieur à environ 45°C ;
et dans laquelle, après la pulvérisation de l'adhésif et son refroidissement à la température ambiante, l'adhésif conserve une liquidité suffisante pendant suffisamment longtemps pour mouiller ou pénétrer un substrat ou une couche pour former un collage résistant à l'humidité.

2. Adhésif selon la revendication 1, dans lequel l'adhésif, lorsqu'il est refroidi, présente une résistance au décollement inférieure à 50 g pendant au moins 5 minutes, puis présente par la suite une résistance au décollement dépassant 100 g.

3. Adhésif selon la revendication 1 ou 2, dans lequel le copolymère séquencé thermoplastique comprend un copolymère séquencé A-B-A possédant un bloc médian de polyisoprène ayant une masse moléculaire d'environ 100 000 à 150 000 et une teneur en styrène d'environ 14 à 30% en poids, la résine poissante étant de préférence une résine poissante aliphatique ;
ou bien la composition de copolymère séquencé thermoplastique comprend un copolymère séquencé A-B-A possédant un bloc médian de polybutadiène ayant une masse moléculaire d'environ 50 000 à 120 000 et une teneur en styrène d'environ 20 à 35% en poids, l'agent poissant étant de préférence un terpène styréné ;
ou encore le copolymère séquencé comprend un mélange de polymère styrène-isoprène-styrène et de copolymère styrène-butadiène-styrène, dans un rapport pondéral d'environ 2 à 5:1, le plastifiant comprenant de préférence un sulfonamide aromatique, et
l'agent poissant comprenant de préférence un dérivé de colophane de bois, en particulier un ester de pentaérythritol d'un acide résinique.

4. Adhésif selon la revendication 1, dans lequel la résine poissante est une résine poissante aliphatique, en particulier une résine poissante aliphatique-aromatique.

5. Adhésif selon l'une quelconque des revendications 1-4, dans lequel le plastifiant possède un point de ramollissement d'au moins environ 65°C.

6. Adhésif selon l'une quelconque des revendications 1-5, dans lequel le plastifiant comprend un ester aliphatique, en particulier cycloaliphatique ou aromatique, d'un acide benzènedicarboxylique, en particulier d'acide 1,2-benzènedicar-boxylique, le plastifiant étant de préférence le phtalate de dicyclohexyle.

7. Adhésif selon l'une quelconque des revendications 1-5, dans lequel le plastifiant comprend un ester d'acide carboxylique aromatique d'un composé polyhydroxylé comportant 1 à 10 groupes hydroxyle, en particulier un ester d'éthylèneglycol, de glycérine, de néopentylglycol, de pentaérythritol, de glucose, de saccharose ou d'un mélange de ceux-ci, et d'acide benzoïque ou d'acide 4-méthylbenzoïque.

8. Adhésif selon l'une quelconque des revendications 1-5, dans lequel le plastifiant comprend un phosphate tri-substitué ou un sulfonamide aromatique.

9. Adhésif selon l'une quelconque des revendications 1-8, qui comprend, en outre, un tensioactif, en particulier un tensioactif non ionique.

10. Adhésif selon l'une quelconque des revendications 1-9, comprenant :
(a) environ 10 à 40 parties en poids d'un copolymère séquencé thermoplastique A-B-A, dans lequel chaque A comprend un bloc de polystyrène et chaque B comprend un bloc de butadiène caoutchouteux ayant une masse moléculaire d'environ 50 000 à 120 000 et une teneur en styrène d'environ 20 à 35% en poids, ou un bloc d'isoprène caoutchouteux ayant une masse moléculaire d'environ 100 000 à 150 000 et une teneur en styrène d'environ 14 à 30% en poids ;
(b) environ 30 à 80 parties en poids d'un ester de colophane ; et
(c) environ 5 à 40 parties en poids d'un composé plas-tifiant aromatique comprenant du phtalate de dicyclohexyle ou du 1,4-dicyclohexanediméthanoldibenzoate ;
dans lequel l'adhésif pulvérisé conserve, après refroidissement, une liquidité suffisante pour mouiller ou pénétrer entièrement un substrat.

11. Article résistant à un décollage sous l'action de l'humidité, comprenant un substrat collé à une seconde couche par un adhésif à pulvériser selon l'une quelconque des revendications 1-10, dans lequel, après la pulvérisation et le refroidissement de l'adhésif à la température ambiante, l'adhésif conserve une liquidité suffisante pendant suffisamment longtemps pour mouiller ou pénétrer le substrat ou la seconde couche, pour former un collage résistant à l'humidité.

12. Article selon la revendication 11, dans lequel le substrat comprend un film et la seconde couche comprend un tissu non tissé, et le tissu est emprisonné par l'adhésif, le film de substrat comprenant de préférence un film de polyéthylène, un film de polypropylène, un film de nylon, un film de poly(chlorure de vinylidène), un film de poly(chlorure de vinyle) ou un film de polyester.

13. Article selon la revendication 11, dans lequel le substrat est une masse de libres absorbantes, la seconde couche est une enveloppe en tissu ouate et le substrat et la seconde couche sont tous deux emprisonnés par l'adhésif.

14. Couche-culotte jetable selon la revendication 11 ou 12, comprenant une couche de non tissé collée sur une couche de film par l'intermédiaire d'un adhésif à pulvériser, ladite couche-culotte jetable résistant à un décollage sous l'action de l'humidité, ledit adhésif comprenant :
(a) environ 10 à 40 parties en poids d'un copolymère séquencé thermoplastique A-B-A, dans lequel chaque A comprend un bloc de polystyrène et chaque B comprend un bloc médian de butadiène caoutchouteux, dans lequel la masse mo-léculaire du polymère est de 50 000 à 120 000 et le polymère comprend de 20 à 35% en poids de polystyrène, ou un bloc médian d'isoprène caoutchouteux, dans lequel la masse moléculaire du polymère est de 100 000 à 150 000 et la teneur en styrène est d'environ 14 à 30% en poids ;
(b) environ 30 à 80 parties en poids d'une résine poissante aliphatique ou aliphatique-aromatique ; et
(c) environ 5 à 40 parties en poids d'un composé plastifiant aromatique comprenant (i) un ester cycloaliphatique ou aromatique d'un composé acide 1,2-benzènedicarboxylique ou (ii) un ester d'acide benzoïque d'un diol aliphatique ou cycloaliphatique en C₂-C₁₂ ayant un point de ramollissement supérieur à environ 45°C, en particulier du 1,4-dicyclohexa-nediméthanoldibenzoate ;
dans laquelle l'adhésif pulvérisé conserve, après refroidissement à la température ambiante, une liquidité suffisante pour pénétrer la couche de non tissé, et au moins une partie du non tissé est emprisonnée et noyée dans l'adhésif à pulvériser.

15. Couche interne absorbante, pour couche-culotte jetable, résistant à un décollage sous l'action de l'humidité, ladite couche comprenant une masse cellulosique absorbante fibreuse ayant une enveloppe en tissu ouate collée à la masse cellulosique par l'intermédiaire d'un adhésif selon l'une quelconque des revendications 1-10 pulvérisé sur l'enveloppe en tissu ouate, ledit adhésif comprenant :
(a) environ 10 à 40 parties en poids d'un copolymère séquencé thermoplastique A-B-A, dans lequel chaque A comprend un bloc de polystyrène et chaque B comprend un bloc médian de butadiène caoutchouteux ayant une masse moléculaire d'environ 50 000 à 120 000 et une teneur en styrène d'environ 20 à 35% en poids, ou un bloc d'isoprène caoutchouteux ayant une masse moléculaire d'environ 100 000 à 150 000 et une teneur en styrène d'environ 14 à 30% en poids ;
(b) environ 30-80 parties en poids d'un dérivé de colophane poissant, d'une résine poissante aliphatique ou d'une résine poissante mixte aliphatique-aromatique, en particulier un agent poissant terpène styréné ; et
(c) environ 5 à 40 parties en poids d'un plastifiant aromatique comprenant (i) un ester d'un alcool cyclique aliphatique, en particulier le cyclohexanol, ou d'un alcool aromatique, en particulier le phénol, d'un composé acide benzènedicarboxylique, en particulier d'acide 1,2-benzènedicarboxylique, ou (ii) un ester d'acide benzoïque d'un diol aliphatique ou cycloaliphatique en C₂-C₁₂ ayant un point de ramollissement supérieur à environ 45°C ;
dans laquelle l'adhésif pulvérisé conserve, après refroidissement, une liquidité suffisante pendant au moins 5 minutes pour pénétrer l'enveloppe de tissu ouate et se coller à la masse cellulosique.
